# FASCICULE DE BREVET EUROPEEN

(11) **EP 1 189 587 B1**
(45) Date de publication et mention de la délivrance du brevet: **12.10.2005**
(21) Numéro de dépôt: 01927989.2
(22) Date de dépôt: 18.04.2001
(51) Int. Cl.: A61K 7/48, A61K 9/70

(54) **ARTICLE COSMETIQUE OU DERMATOLOGIQUE COMPRENANT UNE COMPOSITION D'HYDROCOLLO DE LYOPHILISE**
ARTIKEL FÜR DIE KOSMETIK ODER DIE DERMATOLOGIE, DER EINE GEFRIERGETROCKNETE HYDROKOLLOIDZUSAMMENSETZUNG ENTHÄLT
COSMETIC OR DERMATOLOGICAL PRODUCT COMPRISING A FREEZE-DRIED HYDROCOLLOID COMPOSITION

(30) Priorité: 28.04.2000 FR 0005435
(43) Date de publication de la demande: 27.03.2002
(73) Titulaire: LYOFAL, 13300 Salon de Province (FR)
(72) Inventeur: DECLOMESNIL, Sophie, F-13100 Aix en Provence (FR); ALLEMAND, Jean-Luc, F-13330 Pelissanne (FR)
(74) Mandataire: Domange, Maxime
(86) Numéro de dépôt international: PCT/FR2001/001185
(87) Numéro de publication internationale: WO 2001/082886

(56) Documents cités:
- EP-A- 0 998 949
- FR-A- 2 649 318
- US-A- 4 390 519
- US-A- 4 997 425
- US-A- 5 856 359
- DATABASE WPI Week 9545 Derwent Publications Ltd., London, GB; AN 1995-347501 XP002156411 & JP 07 236688 A (TERUMO CORP.), 12 septembre 1995 (1995-09-12)
- DATABASE CHEMICAL ABSTRACTS [en ligne] STN; abrégé 131: 262 678, XP002156410 & JP 11 276571 A (KATAKURA CHIKKARIN CO., LTD) 12 octobre 1999 (1999-10-12)

## Description

La présente invention concerne un article cosmétique ou dermatologique apte à être appliqué sur la peau.

Un article selon l'invention comprend un substrat solide sur lequel est déposée une composition à activité cosmétique ou dermatologique. Ce type d'article appliqué sur la peau est communément dénommé "patch".

La présente invention concerne également un procédé de fabrication de ce type d'article.

L'invention vise tout particulièrement les articles ou patchs à action hydratante, nourrissante, restructurante, décontractante ou traitante. De tels patchs s'appliquent sur une quelconque partie du corps, et notamment sur le visage.

Il existe des patchs de soin occlusifs que l'on applique en général plusieurs heures, voire toute une nuit, pour administrer dans l'épiderme un agent actif. Ces patchs comprennent un substrat solide revêtu d'un produit actif et présentent un pouvoir adhésif sur la peau très élevé. Lors de leur enlèvement, ces patchs sont très agressifs et traumatisants pour la peau.

Ainsi, on connaît dans US 5 856 359 un patch pharmaceutique à action transdermique, dans lequel une composition pharmaceutique sous forme d'un gel renfermant de la thyroxine est appliquée sur un support tel qu'un film plastique de polyester que l'on colle à la peau.

On connaît également des patchs à base de substrats constitués de polymères hydrosolubles biorésorbables, notamment à base de polymères polylactides ou polyglycolides lyophylisés. Ces patchs dont les substrats se résorbent n'ont aucune action occlusive. Des patchs de ce type sont décrits dans les abrégés des publications Derwent citées dans le rapport de recherche de la demande de brevet en France n° 00 0543500 servant de priorité à la présente demande de brevet, sous les références XP 002156411 au nom de la société Terumo Corp.

On connaît également dans US 4 997 425 et US 4 390 519 des pansements hémostatiques ou cicatrisants du type compresse, comprenant un polymère réticulé et donc non hydrodispersible, destinés à absorber les exudats de plaies et à les protéger des agressions externes tout en les maintenant dans un environnement humide résultant de la lymphe de la plaie, ce qui favorise la cicatrisation.

Le but de la présente invention est de fournir un nouveau type d'article cosmétique ou dermatologique comprenant un substrat à action occlusive adhérant parfaitement à la peau, mais sans adhésif, facilement manipulable avant et pendant les soins, et facilement retirable après la durée des soins.

Un autre but de la présente invention est de fournir un article cosmétique ou dermatologique de type patch, dont l'action cosmétique ou dermatologique soit rapide et efficace, notamment une action hydratante, nourrissante, restructurante, décontractante et/ou traitante.

Un autre but de la présente invention est de fournir un article cosmétique ou dermatologique de type patch qui puisse être appliqué sur tout type de peau, même les plus sensibles et qui puisse délivrer tout type de principe actif.

Un autre but de la présente invention est de fournir un article dermatologique de type patch, qui puisse être conservé en garantissant la stabilité de ses composants avant utilisation du point de vue microbiologique et du point de vue de l'activité des principes actifs eux-mêmes, sans ajout de conservateur.

Pour ce faire, la présente invention fournit un article cosmétique ou dermatologique apte à être appliqué sur la peau, qui comprend une composition à action cosmétique ou dermatologique lyophilisée adhérant à un substrat solide, ladite composition comprenant une matrice d'un agent texturant hydrophile, de préférence non réticulé, renfermant de préférence au moins un principe actif, ladite composition étant hydrosoluble ou hydrodispersible après réhydratation. Ainsi, l'action cosmétique ou dermatologique de l'article selon l'invention peut être obtenue rapidement, notamment en quelques minutes, ou résulter d'une mise en contact plus longue, par exemple une heure.

Un article selon la présente invention doit être réhydraté au moment de l'application sur la peau, soit par trempage dans une solution aqueuse, soit par pulvérisation d'une solution aqueuse sur ladite composition de revêtement, soit par dépôt de l'article sous forme lyophilisée sur une peau humide, notamment à la sortie d'un bain. La réhydratation se fait au moyen d'un liquide aqueux qui peut être soit de l'eau proprement dite, soit une substance aqueuse elle-même contenant un produit actif renforçant l'action du principe actif inclus dans l'article. Une fois réhydratée la composition lyophilisée présente une viscosité telle qu'elle permet l'adhérence parfaite à la peau mais sans adhésif, de sorte que ledit substrat peut être facilement retiré après la durée du soin. Après réhydratation ladite composition forme une solution visqueuse ou un gel et adhère donc à la peau de par sa viscosité, mais l'article est dépourvu de tout adhésif.

Lorsque la matrice lyophilisée est réhydratée puis mise au contact de la peau, elle peut avoir une action sur la peau de par les composants constitutifs de l'agent texturant hydrophile ou/et de préférence de un ou plusieurs principes actifs qu'elle contient, ladite action pouvant se faire soit par diffusion à l'intérieur de la peau, c'est-à-dire par transdermie ou par simple effet de contact à la surface de la peau.

La composition à action cosmétique ou dermatologique de par sa nature lyophilisée, présente une structure expansée poreuse provenant dudit agent texturant hydrophile encore appelé "hydrocolloïde" qui forme une matrice au sein de laquelle ledit principe actif se trouve renfermé et uniformément dispersé.

Ladite composition à action cosmétique ou dermatologique est dite "hydrosoluble ou hydrodispersible", c'est-à-dire qu'elle peut se dissoudre en phase aqueuse ou à tout le moins se disperser, ce qui lui permet de se déliter et pénétrer le cas échéant la peau après application, notamment du fait que ledit agent de texture n'est pas un polymère réticulé.

L'article selon l'invention peut être appliqué sur la peau après réhydratation, soit de manière statique, soit en lui appliquant un mouvement relatif par rapport à la peau, ayant un effet de massage, de manière à étaler ladite composition réhydratée à la surface de la peau et à faciliter sa pénétration éventuelle. Ce massage permet un étalement de la composition active sur une plus grande surface que la surface du substrat.

Il y a lieu de noter que ledit substrat, outre son effet de support matériel de ladite composition à activité cosmétique ou dermatologique, présente un effet occlusif vis à vis des principes actifs et des agents texturant constitutifs favorisant leur diffusion en direction de la peau d'une part, et d'autre part de par sa texture et sa structure, il présente un effet favorisant l'accrochage et l'adhérence de la composition lyophilisée à la surface dudit substrat.

A cet égard, dans un mode préférentiel de réalisation; ledit substrat présente une structure poreuse permettant une imprégnation de ladite composition dans ledit substrat, au moins à proximité de la surface dudit substrat.

La structure poreuse du substrat permet après imprégnation partielle, c'est-à-dire au moins superficielle, du substrat par ladite composition, d'obtenir une bonne adhésion de la composition sur le substrat suite à la lyophilisation de la composition préalablement appliquée sur le substrat, car l'agent texturant est ainsi enchevêtré ou accroché avec la matière constitutive du substrat en surface de celui-ci.

Le substrat solide de l'article selon l'invention, constitue un support qui peut se présenter sous forme d'un matériau en feuille tissé ou non tissé, d'un filet ou même d'une feuille ou d'un film perforé(e) ou encore d'une mousse synthétique cellulaire à cellules ouvertes, notamment à base de polyuréthane.

La composition lyophilisée est intégrée à la surface du substrat mais peut également être en outre noyée à l'intérieur du substrat par les propriétés absorbantes du substrat. On peut également envisager que le substrat est entièrement noyé à l'intérieur de ladite composition lyophilisée.

D'une manière générale, la fonction du substrat est multiple : principalement, il facilite la manipulation de l'article, d'autant plus que les lyophilisats sont hydroscopiques. Grâce au substrat, même avec une composition lyophilisée contenant une matrice très fine, l'article reste ainsi manipulable. Ledit substrat facilite également le positionnement de l'article humidifié sur la peau lors du traitement et la forme du substrat permet, comme il sera explicité ultérieurement, de suivre le profil de la surface de la peau.

Ainsi accrochée sur le substrat, et de par sa viscosité, ladite composition à action cosmétique ou dermatologique, bien qu'elle comporte des composants aqueux ou hydrophiles, ne coule pas après réhydratation. Comme mentionné précédemment, le substrat apporte aussi une barrière occlusive accentuant la diffusion de la matrice et plus particulièrement du principe actif vers et dans le derme, ce qui amplifie l'action du soin de façon remarquable. A la fin de la durée du soin, le substrat est retiré, toute la matrice ou une partie seulement selon la durée du soin a disparu lors de l'application par transfert sur la peau.

L'article selon l'invention, lors de son enlèvement, n'est ni agressif, ni traumatisant pour la peau, de par l'absence d'adhésif.

La conservation de la composition sous forme lyophilisée permet d'obtenir un produit stable du point de vue microbiologique et du point de l'activité des principes actifs sans ajout de conservateur. Elle permet également d'obtenir une large gamme d'articles très actifs pour tout type de peaux, même les plus sensibles.

Enfin, la lyophilisation permet de stabiliser même les principes actifs les plus thermolabiles et/ou les plus hydrolabiles. La lyophilisation confère également une structure poreuse expansée à ladite matrice qui se réhydrate instantanément et confère à l'article un toucher remarquablement doux ainsi qu'une souplesse qui facilité son utilisation.

Avantageusement, ledit substrat est un substrat fibreux, de préférence encore, ledit substrat fibreux est constitué d'une nappe fibreuse non tissée.

Le substrat peut être réalisé à partir de fibres d'origine végétale, notamment des fibres de cellulose ou de coton ou des dérivés de ces derniers ; il peut s'agir notamment d'un feutre.

De préférence, le substrat est réalisé à partir de fibres d'origine synthétique, de préférence de polyoléfine tels que du polypropylène, des polyamides, des polyesters ou des viscoses.

Dans un autre mode de réalisation, ledit substrat peut être constitué par une mousse cellulaire, notamment à base de polyuréthane.

Dans un mode particulier de réalisation, le substrat se présente sous forme d'une feuille d'épaisseur de 100 µm à 3 cm, de préférence de 0,2 à 5 mm d'épaisseur.

De même, la composition lyophilisée selon la présente invention présente une épaisseur de 100 µm à 10 mm, de préférence de 0,1 à 5 mm, de préférence encore de 0,5 à 3 mm.

Ledit substrat peut avoir une forme plane, souple ou semi-rigide, de préférence adaptée à la forme de la partie du corps sur laquelle il est destiné à être appliqué.

Dans un mode de réalisation avantageux, ledit substrat présente une texture avec des pores ou creux de surface de plus grande taille de 100 µm à 2 mm au moins sur la surface sur laquelle est appliquée ladite composition à activité cosmétique ou dermatologique.

Dans un mode de réalisation particulier, ledit substrat présente une structure de surface texturée, gaufrée, rainurée, avec des creux et des bosses, et plus particulièrement avec une trame régulière ajoutée en surface constituée de préférence de côtes ou de picots en élévation par rapport à une surface plane. Cette texture favorise l'efficacité des massages éventuels de la peau lorsque l'on applique ledit article en mouvement sur la peau.

Ainsi, un substrat selon l'invention peut être constitué par une nappe fibreuse non tissée en feuille revêtue par des picots en matériau plastique ou une trame constituée d'un quadrillage de côtes en matériau plastique de type polypropylène par exemple, fixée en surface d'une nappe fibreuse ou d'un feutre .

Dans un mode de réalisation, ladite trame délimite des creux régulièrement espacés de 100 µm à 2 mm, et peut être formée dans la même matière constitutive que la masse dudit substrat.

On comprend que la surface du substrat présentant le texturage, gaufrage, rainurage ou la trame mentionné ci-dessus, est elle-même revêtue de ladite composition lyophilisée et que l'effet massant se produit au fur et à mesure que la composition lyophilisée réhydratée se délite et se disperse à la surface de la peau au cours du massage.

Comme mentionné précédemment, avantageusement ledit article est un masque et ledit substrat a une forme adaptée pour recouvrir le visage ou une partie du visage, notamment les yeux, les lèves, le nez ou les joues.

Dans un mode préférentiel de réalisation, ledit agent texturant est un polymère hydrocolloïde non réticulé d'origine naturelle ou synthétique.

Plus particulièrement, ledit agent texturant est choisi parmi :
- les agents de texture naturels d'origine végétale tels que la gomme de caroube, la gomme de guar, les pectines, les alginates, les carraghénanes, l'agar-agar, la gomme arabique ;
- les agents d'origine microbienne tels que la gomme de xanthane, la gomme de gellane, l'acide hyaluronique, le dextrane ;
- les agents d'origine animale tels que la gélatine, le collagène, le chitosane ; et
- les agents de texture semi-synthétiques comme la cellulose ou synthétique comme les polymères hydrogels à base de polymères non bio-résorbables du type polyacrylique ou polyacrylamide.

L'agent de texture hydrophile lyophilisé sert de vecteur à l'actif, donne de la consistance à la formule cosmétique ou dermatologique et peut également jouer un rôle d'actif hydratatant, filmogène ou humectant.

Dans un mode de réalisation, on préfère pour ce faire utiliser plus particulièrement comme agent texturant l'acide hyaluronique, le chitosane et ses dérivés comme le chitosane succinamide, les alginates ou le collagène marin.

De façon à optimiser les conditions d'adhérence de la composition lyophilisée à la surface du substrat d'une part, et d'autre part son adhésion à la peau après réhydratation, on utilise un agent texturant hydrophile tel que après réhydratation avec de préférence 0,05 g/cm² à 0,15 g/cm² d'eau, ladite composition lyophilisée réhydratée forme une solution visqueuse ou un gel qui présente une viscosité de 400 à 5000 centipoises, de préférence 500 à 2000 centipoises.

Dans un mode de réalisation particulier de l'invention, ladite composition lyophilisée est appliquée à la surface dudit substrat dans un grammage de 5 mg/cm² à 1000 mg/cm², de préférence 20 mg/cm² à 200 mg/cm².

Le composé actif incorporé dans l'agent de texture hydrophile peut être ajouté à un taux variable, notamment le rapport massique dudit principe actif par rapport au poids total de la composition lyophilisée peut aller de 0,05 à 5.

Ledit principe actif peut être un agent hydrophile et donc hydrosoluble ou lipophile et donc liposoluble.

Ladite composition peut se présenter sous forme d'une émulsion dans l'eau ou une émulsion d'eau dans l'huile, ledit principe actif étant alors dissout soit dans la phase grasse, soit dans la phase aqueuse de ladite émulsion.

Lorsque ladite composition se présente sous forme d'une émulsion, elle comprend également un agent tensioactif pour favoriser l'émulsion.

Lorsque la composition se présente sous forme d'une émulsion d'huile dans l'eau, le principe actif est alors de préférence un principe actif lipophile et donc liposoluble dans ladite phase grasse de l'émulsion. Dans ce cas, en général, le rapport pondéral dudit principe actif par rapport au poids total de la composition lyophilisée est plus particulièrement compris entre 5 et 50%, en général de l'ordre de 10 à 15%.

Les principes actifs incorporés dans l'agent de texture hydrophile peuvent être très divers, il peut s'agir notamment d'un principe actif habituellement utilisé dans les domaines cosmétiques et/ou dermatologiques et en particulier, un principe actif choisi parmi un agent antioxydant à action anti-radicalaire, un agent dépigmentant, un agent liporégulateur, un agent anti-acnéique, un agent anti séborrhéique, un agent anti-vieillissement, un agent adoucissant, un agent antirides, un agent anti-inflammatoire, un agent cicatrisant, un agent hydratant, un agent anti-bactérien, un agent anti-fongique, un agent filtre anti-UV, une protéine, un acide aminé, une huile.

Plus particulièrement, ledit principe actif est choisi parmi une huile riche en acide gras polyinsaturé, un phytostérol, une céramide, un extrait de plantes ou un extrait d'algues, notamment un polysaccharide d'origine bactérienne ou végétale.

Dans un mode de réalisation particulier, ledit agent texturant présente une action hydratante et ledit principe actif une action traitante, éventuellement aussi hydratante.

Dans un mode de réalisation particulier, ledit article selon l'invention est conditionné dans une pochette souple ou semi-rigide ou dans une coque rigide ou semi-rigide, ladite coque étant hermétiquement scellée par un opercule, de préférence un film métallique, l'article se trouvant dans une atmosphère sous vide ou de gaz neutre tel que CO₂ ou N₂.

La présente invention a également pour objet un procédé de fabrication d'un article selon lequel on réalise les étapes dans lesquelles :
- on applique une solution de ladite composition d'un agent texturant hydrophile et de préférence, d'au moins un agent actif, à l'état liquide, sur ledit substrat, puis
- on lyophilise ladite composition une fois celle-ci appliquée sur ledit substrat.

L'étape de lyophilisation intervenant après l'application de ladite composition à activité cosmétique ou dermatologique sur ledit substrat, est importante pour obtenir une bonne adhérence de ladite composition à la surface du substrat, car la lyophilisation intervient après qu'une certaine imprégnation de la composition liquide ait pu se produire au moins en superficie dudit substrat.

De préférence, on utilise une solution de ladite composition à une concentration de 0,1 à 5% de préférence de 0,5 à 2% en poids dudit agent texturant hydrophile, celui-ci présentant une viscosité de préférence de 200 à 5000 centipoises, de préférence de 400 à 1000 centipoises. La viscosité de ladite composition dépend essentiellement du poids moléculaire et de la concentration de l'agent texturant hydrophile mis en oeuvre.

Avantageusement, selon le procédé de l'invention, on réalise les étapes dans lesquelles :
- ledit substrat est prédécoupé de préférence à une forme géométrique définie en fonction de la zone du corps à laquelle l'article est destiné,
- ledit substrat et ladite matrice sont introduits dans une coque support,
- on réalise la lyophilisation dudit article dans ladite coque.

Ledit article prend ainsi la forme de ladite coque dans laquelle il a été lyophilisé.

Deux procédés de fabrication des articles selon l'invention sont possible. Selon un premier mode de réalisation, le substrat est déposé sur un support puis enduit par ladite matrice, ensuite celle-ci est séchée par lyophilisation, puis le substrat est découpé à la forme souhaitée. L'article lyophilisé est ensuite conditionné à l'intérieur d'une pochette ou d'un blister étanche.

On utilise préférentiellement un matériau de conditionnement étanche à l'oxygène et à la vapeur d'eau. Il s'agit notamment d'un matériau en matière plastique. Le conditionnement peut être réalisé sous vide ou sous atmosphère de gaz neutre.

Selon un second mode de réalisation, le substrat est prédécoupé et introduit dans une coque support qui peut aussi servir de conditionnement de l'article ultérieurement, et dont la forme géométrique est définie en fonction du corps ou du visage auquel l'article est destiné. Dans ce cas, soit le substrat est déposé au fond de ladite coque, puis enduit par ladite matrice, soit ladite matrice est tout d'abord coulée dans ladite coque et le substrat est déposé par-dessus ladite matrice. On peut également noyer ledit substrat dans ladite matrice.

Dans les deux cas, la lyophilisation intervient lorsque ledit substrat et ladite matrice sont introduits dans ladite coque et le substrat prend la forme de la coque dans laquelle il a été lyophilisé, notamment par mémoire de forme.

Dans un autre mode de réalisation, l'article lyophilisé est démoulé et conditionné à l'intérieur d'une pochette ou d'un blister étanche. On utilise alors préférentiellement un matériau de conditionnement étanche à l'oxygène et à la vapeur d'eau, le conditionnement étant réalisé sous vide ou sous atmosphère de gaz neutre, notamment CO₂ ou N₂.

Quel que soit le procédé de fabrication utilisé, les articles selon la présente invention peuvent prendre la forme d'un masque pour le visage, d'un contour de l'oeil, d'un contour des lèvres, de la zone entre le nez et la lèvre supérieure, du coin externe de l'oeil pour traiter les rides dites de patte d'oie, des joues ou encore du front. Les articles selon l'invention peuvent également prendre toute autre forme selon la zone du corps à traiter. En général, la taille des articles selon l'invention est comprise entre 0,25 cm² et 650 cm², de préférence entre 5 et 100 cm².

La lyophilisation est une technique douce de séchage par le froid et le vide, connue de l'homme de l'art, qui permet de conserver le principe actif sans l'altérer.

Dans un mode particulier de réalisation de la lyophilisation, on refroidit le mélange jusqu'à une température inférieure à - 10°C, de préférence comprise entre - 20 et - 80°C, pour le congeler ou le surgeler, de préférence pendant une durée d'au moins une heure, par exemple de 2 à 10 heures. On place ensuite le produit gelé obtenu sous un vide correspondant à une pression absolue, de préférence supérieure à 10 Pa, de préférence inférieure à 100 Pa, en maintenant le produit à une température inférieure à - 5°C, de préférence de -15 à -40°C pour déshydrater le produit gelé par sublimation, de préférence pendant une durée de plusieurs heures, notamment une durée voisine de 10 à 50 heures. Puis, on provoque une déshydratation secondaire (désorption) par chauffage progressif du produit jusqu'à une température finale supérieure à 0°C et inférieure à 50°C, de préférence de 20 à 50°C, de préférence encore pendant une durée de plusieurs heures, notamment de 5 à 30 heures.

D'autres caractéristiques et avantages de la présente invention apparaîtront à la lumière des exemples de réalisation qui vont suivre.
La figure 1 est une représentation schématique d'un article selon l'invention, avant application (fig. 1A) et après application sur la peau en fin d'utilisation (fig. 1B).
Les figures 2 et 3 représentent des formes de substrat fibreux pour constituer un masque de visage (fig.2) ou des contours des yeux (fig. 3 et 4).

### Exemple 1 - Masque de beauté lyophilisé sur support fibreux.

On réalise une composition cosmétique comprenant le mélange suivant dans de l'eau osmosée avec les teneurs pondérales suivantes :
- chitosane succinamide à 1%
- acide ascorbique pur à 0,1%

La composition présente une viscosité de 1000 centipoises. Elle est coulée à température ambiante dans une coque thermoformée en PVC dans laquelle on a préalablement déposé au fond une nappe de fibres non tissées en polypropylène constituant une feuille de 200 µm d'épaisseur. La composition est coulée et dépasse en surface sur une épaisseur moyenne de 1 mm.

Après coulage la coque est placée dans l'enceinte d'un lyophilisateur, le patch est séché par lyophilisation. Les paramètres du procédé de lyophilisation pour cet exemple sont :
- température de congélation - 40 ° C,
- température de sublimation - 20 °C,
- température de dessiccation secondaire + 30° C,
- vide partiel 400 µbars.

Après lyophilisation la coque est fermée par un opercule en aluminium thermoscellé.

Après démoulage par l'utilisatrice, le patch est humecté et appliqué sur le visage pendant 10 minutes ou plus. Un tel patch a une application hydratante, filmogène et anti-oxydante.

Le chitosane en tant qu"hydrocolloïde a un haut pouvoir de rétention d'eau et permet de maintenir la peau humide pendant toute la durée d'application du patch, qui peut aller jusqu'à une heure ou deux. La fonction de l'hydrocolloïde est également de permettre l'emprisonnement du principe actif et sa libération progressive et son adhérence aussi bien au substrat qu'à la peau de par ses propriétés gélifiantes. L'hydrocolloïde permet également de diluer le principe actif fortement dosé. Le principe actif est conservé en matrice lyophilisée et devient actif après libération lorsqu'on humidifie ladite matrice.

L'article lyophilisé qui se présente sous forme sèche et s'active au contact de l'eau permet de conserver le principe actif stable sans ajout de conservateur jusqu'au moment de son utilisation. On évite ainsi les risques de perte d'activité au cours de la conservation.

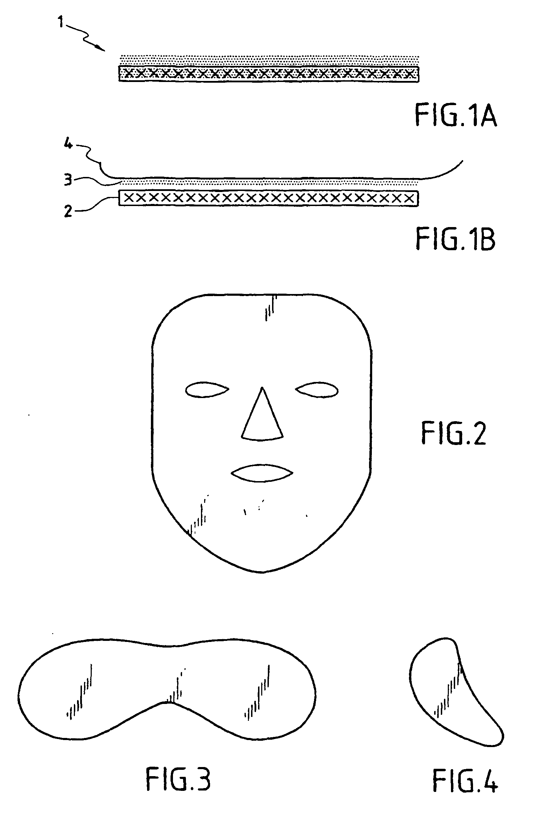

Sur la figure 1A on a représenté l'article lyophilisé 1 dont le substrat 2 est partiellement imprégné de ladite composition lyophilisée 3 comprenant l'hydrocolloïde et le principe actif. Après humidification de l'article avec de l'eau et application sur la peau, l'hydrocolloïde et le principe actif migrent et se déposent sur la peau, comme représenté sur la figure 1B où une partie de la composition est représentée en surface de la peau.

### Exemple 2 - Emulsion pour peau sèche

On a réalisé une émulsion avec la composition décrite dans le tableau 1 ci-après, dans lequel :
- Les composants n° 1 à 3 constituent la phase grasse de l'émulsion.
- Le composé n° 4 constitue l'agent tensioactif émulsifiant.
- Les composés n° 6 et 7 constituent les principes actifs et sont des agents anti-déshydratants.
- Le composé n° 9 constitue l'hydrocolloïde.

La viscosité de la composition est de 600 centipoises.

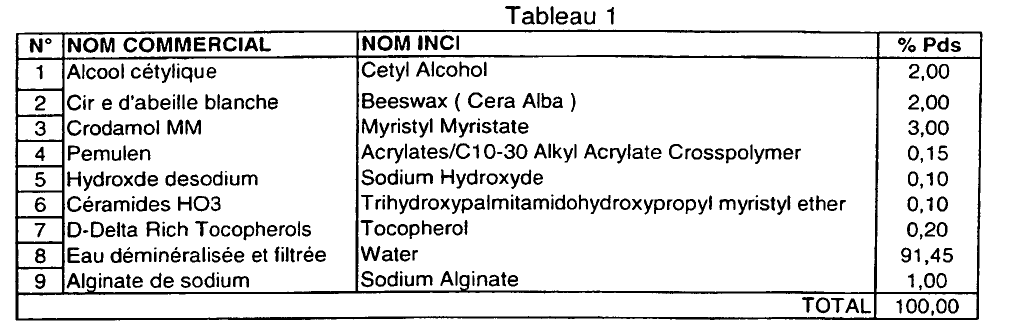

### Exemple 3 - Emulsion amincissante

On a réalisé l'émulsion avec les composés suivants dans les teneurs pondérales suivantes indiquées dans le tableau 2 ci-après dans lequel :
- Les composants n° 1 à 3 constituent la phase grasse de l'émulsion.
- Le composé n° 4 constitue l'agent tensioactif émulsifiant.
- Le composé n° 5 est un extrait de plante à action amincissante et le composé n° 6 est un agent solubilisant du composé 5.
- Les composés n° 8 et 9 constituent des hydrocolloïdes.
- Le composé n° 10 a pour fonction de neutraliser le Carbopol.

La viscosité de cette composition est de 600 centipoises.

**Tableau 2**

| **N°** | **NOM COMMERCIAL** | **NOM INCI** | **% Pds** |
|---|---|---|---|
| 1 | Alcool cétylique | Cetyl Alcohol | 2,00 |
| 2 | Cire d'abeille blanche | Beeswax (Cera Alba) | 2,00 |
| 3 | Crodamol MM | Myristyl Myristate | 3,00 |
| 4 | Pemulen | Acrylates/C10-30 Alkyl Acrylate Crosspolymer | 0,15 |
| 5 | Coleus barbatus | Coleus barbatus extract | 0,15 |
| 6 | Transcutol CG | Ethoxydiglycol | 2,25 |
| 7 | Eau déminéralisée et filtrée | Water | 88,85 |
| 8 | Alginate de sodium | Sodium Alginate | 1,00 |
| 9 | Carbopol (Polymère polyacrylique) | Polyacrylic Polymer | 0,50 |
| 10 | Hydroxyde de sodium | Sodium Hydroxyde | 0,10 |
| | | TOTAL | 100,00 |

### Exemple 4 - Gel antirides

On a réalisé une formulation cosmétique selon la composition de viscosité de 800 centipoises du tableau 3 dans lequel :
- Le composé 1 est un extrait de plantes accélérateur de renouvellement cellulaire ou à action restructurante.
- Le composé 3 est un agent hydrocolloïde hydratant.

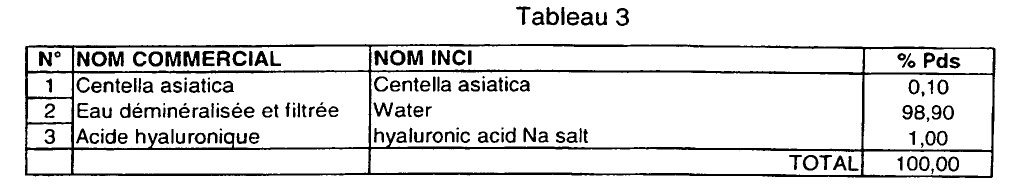

### Exemple 5 - Gel antiradicalaire

On a réalisé une formulation cosmétique selon la composition de viscosité de 1000 centipoises du tableau 4 dans lequel :
- Le composé 1 est un principe actif.
- Le composé 3 est un agent à fonction de parfum.
- Le composé 4 est un hydrocolloïde texturant.

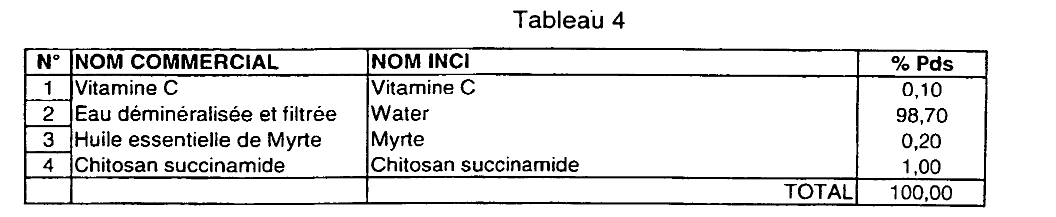

### Exemple 6 - GEL antiinflamatoire pour peau sensible

On a réalisé une formulation cosmétique selon la composition de viscosité de 1000 centipoises du tableau ci-après dans lequel :
- Le composé 1 est un principe actif polysaccharide extrait de le bactérie Altéromonas.

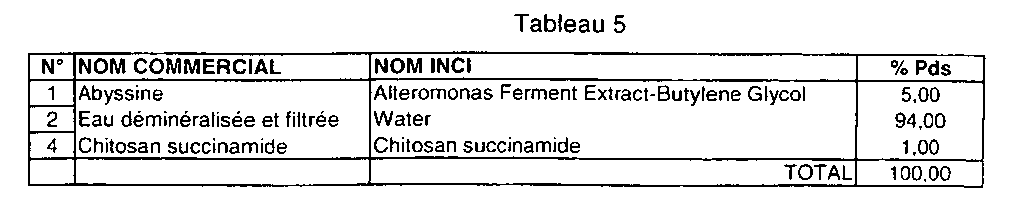

### Exemple 7 - Principes actifs cosmétiques

Dans le tableau 6 suivant, on présente la liste de principes actifs hydrosolubles ou liposolubles avec leur action cosmétique ou dermatologique principale.

**Tableau 6**

| Principes actifs hydrosolubles | Principes actifs liposolubles |
|---|---|
| Lanachrys 2B (minceur) | Rhodystérol (minceur) |
| Centella Asiatica (minceur et renouvellement cellulaire | Coleus Garcina (minceur) |
| Abyssine (anti inflammatoire) | Caféine (minceur) |
| Acide glycyrrhétinique diK (anti inflammatoire) | Acide ursolique (antirides) |
| Acide glycolique (Kératolytique) | Acide hydroxy décanoique (anti sébum) |
| Acide lactique (Kératolytique) | Huile de graine d'Andibora (anti inflammatoire) |
| Alistine (antilipopéroxydation) | Acide Kojique (éclaircissant) |
| Aloe vera gel (anti inflammatoire) | Acide Norhydroguaiarétique |
| Extrait d'Arbutine (anti taches) | Céramide 3 (hydratant) |
| Ectoin (antiagression - protection UV et pollution)) | Céramide 6 (hydratant) |
| Dihydroxy acetone (bronzant) | Triglycérides péroxydés (hydratant) |
| Erthrulose (bronzant) | Butyl méthoxydibenzoylmethane (Filtres solaires) |
| Extrait de mimosa ténuiflora (cicatrisant) | Titane micronisé |
| | Oxyde de zinc micronisé |
| | Acide Ximenique (anti inflammatoire |
| | Octocrylène (Filtre solaire) |
| | Octyl methoxycinnamate (Filtre solaire) |

### Exemple 8 - Substrats

On a appliqué les compositions des exemples 2 à 6 sur des substrats en feuilles non tissés connus comme compresse d'usage médical à base de mélanges de fibres de polyester, viscose, et polyamide présentant les caractéristiques suivantes (normes ISO 9073 et EN 29073) :
- épaisseur : 1 à 3 mm
- poids : 100 à 300 g/m²
- absorption d'eau : 2000 à 3000 g/m²
- volume des pores : 80 à 95%.

La composition après lyophilisation représente une épaisseur de 1 à 3 mm à raison de 20 à 200 mg/cm².

## Revendications

1. Article cosmétique ou dermatologique apte à être appliqué sur la peau, comprenant une composition à action cosmétique ou dermatologique lyophilisée adhérant à un substrat solide, **caractérisé en ce que** ladite composition lyophilisée comprend une matrice à structure expansée poreuse d'un agent texturant hydrophile étant un polymère hydrocolloïde non réticulé, renfermant de préférence au moins un principe actif, ladite composition se présentant sous la forme d'une émulsion d'huile dans l'eau ou une émulsion d'eau dans l'huile, ladite composition lyophilisée étant hydrosoluble ou hydrodispersible après réhydratation.

2. Article selon la revendication 1, **caractérisé en ce que** ledit substrat présente une structure poreuse permettant une imprégnation de ladite composition dans ledit substrat, au moins à proximité de la surface dudit substrat.

3. Article selon l'une des revendications 1 ou 2, **caractérisé en ce que** ledit substrat est un substrat fibreux.

4. Article selon la revendication 3, **caractérisé en ce que** ledit substrat fibreux est constitué d'une nappe fibreuse non tissée.

5. Article selon l'une des revendications 3 ou 4, **caractérisé en ce que** le substrat est réalisé à partir de fibres d'origine synthétique, de préférence des polyoléfines, polyamides, polyesters ou viscoses.

6. Article selon l'une des revendications 1 à 5, **caractérisé en ce que** ledit substrat présente une épaisseur de 100 µm à 3 cm, de préférence de 0, 2 mm à 5 mm.

7. Article selon l'une des revendications 1 à 6, **caractérisé en ce que** ledit substrat a une forme plane souple ou semi-rigide.

8. Article selon l'une des revendications précédentes, **caractérisé en ce que** ledit substrat présente une surface texturée, gaufrée, rainurée, avec des creux et des bosses, ou une trame régulière constituée de préférence de côtes ou de picots en élévation par rapport à une surface plane.

9. Article selon l'une des revendications précédentes, **caractérisé en ce que** ledit substrat présente une texture avec des pores ou creux de surface de plus grande taille de 100 µm à 2mm, au moins sur la surface sur laquelle est appliquée ladite composition.

10. Article selon l'une des revendications précédentes, **caractérisé en ce que**, de préférence, ledit substrat a une forme adaptée à la forme de la partie du corps sur laquelle il est destiné à être appliqué ; de préférence, ledit article est un masque et ledit substrat a une forme adaptée pour recouvrir le visage ou une partie du visage, notamment les yeux, les lèvres, le nez ou les joues.

11. Article selon l'une des revendications précédentes, **caractérisé en ce que** ledit agent texturant est un polymère hydrocolloïde non réticulé d'origine naturelle.

12. Article selon la revendication 11, **caractérisé en ce que** ledit agent texturant est choisi parmi :
- les agents de texture naturels d'origine végétale tels que la gomme de caroube, la gomme de guar, les pectines, les alginates, les carraghénanes, l'agar-agar, la gomme arabique ;
- les agents d'origine microbienne tels que la gomme de xanthane, la gomme de gellane, l'acide hyaluronique, le dextrane ;
- les agents d'origine animale tels que la gélatine, le collagène, le chitosane et ses dérivés; et
- les agents de texture semi-synthétiques comme la cellulose semi-synthétique ou synthétique comme les polymères hydrogels à base de polymères non bio-résorbables du type polyacrylique ou polyacrylamide.

13. Article selon la revendication 12, **caractérisé en ce que** l'agent de texture hydrophile lyophilisé est choisi parmi l'acide hyaluronique, le chitosane et ses dérivés, les alginates ou le collagène marin.

14. Article selon l'une des revendications précédentes, **caractérisé en ce que**, après réhydratation avec de préférence 0,05 g/cm² à 0,15 g/cm² d'eau, ladite composition lyophilisée réhydratée présente une viscosité de 400 à 5000 centipoises, de préférence 500 à 2000 centipoises.

15. Article selon l'une des revendications précédentes, **caractérisé en ce que** ladite composition lyophilisée est appliquée à la surface dudit substrat dans un grammage de 5 mg/cm² à 1000 mg/cm², de préférence 20 mg/cm² à 200 mg/cm².

16. Article selon l'une des revendications précédentes, **caractérisé en ce que** ledit principe actif est un agent hydrophile ou lipophile.

17. Article selon la revendication 16, **caractérisé en ce que** ladite composition se présente sous forme d'une émulsion d'huile dans l'eau ou une émulsion d'eau dans l'huile, ledit principe actif étant dissout, dans la phase grasse ou aqueuse.

18. Article selon la revendication 17, **caractérisé en ce que** ladite composition se présente sous forme d'une émulsion d'huile dans l'eau, ledit principe actif étant liposoluble dans ladite phase grasse de l'émulsion, et ladite composition comprend en outre un agent tensio-actif.

19. Article selon l'une des revendications 16 à 18, **caractérisé en ce que** ledit principe actif est choisi parmi un agent antioxydant à action anti-radicalaire, un agent dépigmentant, un agent liporégulateur, un agent anti-acnéique, un agent anti séborrhéique, un agent anti-vieillissement, un agent adoucissant, un agent antirides, un agent anti-inflammatoire, un agent cicatrisant, un agent hydratant, un agent anti-bactérien, un agent anti-fongique, une vitamine, une protéine, un acide aminé, et une huile.

20. Article selon l'une des revendications 16 à 19, **caractérisé en ce que** ledit principe actif est choisi parmi une huile riche en acide gras polyinsaturé, un phytostérol, une céramide, un extrait de plantes, un extrait d'algues ou un extrait bactérien et une vitamine.

21. Article selon l'une des revendications 1 à 20, **caractérisé en ce que** ledit agent texturant présente une action hydratante et ledit principe actif une action traitante.

22. Article selon l'une des revendications 1 à 21, **caractérisé en ce qu'**il est conditionné dans une pochette souple ou semi-rigide ou dans une coque rigide ou semi-rigide, ladite coque étant hermétiquement scellée par un opercule, de préférence un film métallique, l'article se trouvant dans une atmosphère sous vide ou de gaz neutre.

23. Procédé de fabrication d'un article selon l'une des revendications 1 à 22, **caractérisé en ce qu'**on réalise les étapes dans lesquelles :
- on applique une solution de ladite composition d'un agent texturant hydrophile et de préférence, d'au moins un agent actif, à l'état liquide, sur ledit substrat, puis
- on lyophilise ladite composition une fois celle-ci appliquée sur ledit substrat.

24. Procédé selon la revendication 23, **caractérisé en ce qu'**on utilise une solution aqueuse de ladite composition à une concentration de 0,1 à 5% de préférence de 0,5 à 2% en poids dudit agent texturant hydrophile, celui-ci présentant de préférence une viscosité de 200 à 5000 centipoises, de préférence de 400 à 1000 centipoises.

25. Procédé selon l'une des revendications 23 ou 24, **caractérisé en ce qu'**on réalise les étapes dans lesquelles :
- ledit substrat est prédécoupé, de préférence, selon une forme géométrique définie en fonction de la zone du corps à laquelle l'article est destiné,
- ledit substrat et ladite matrice sont introduits dans une coque support,
- on réalise la lyophilisation dudit article dans ladite coque.

26. Procédé selon la revendication 25, **caractérisé en ce que** ladite coque est utilisée comme conditionnement final de l'article, ladite coque étant revêtue d'un opercule, ledit article étant dans une atmosphère sous vide ou sous gaz neutre.

## Patentansprüche

1. Kosmetischer oder dermatologischer Artikel, der geeignet ist, auf die Haut aufgetragen zu werden, enthaltend eine lyophilisierte, an einem festen Substrat haftende Zusammensetzung mit kosmetischer oder dermatologischer Wirkung, **dadurch gekennzeichnet, daß** die lyophilisierte Zusammensetzung eine Matrix mit ausgedehnter, poröser Struktur eines hydrophilen Strukturfestigers, der ein unvernetztes Hydrokolloid-Polymer ist, umfaßt, die vorzugsweise wenigstens einen wirksamen Bestandteil enthält, wobei die Zusammensetzung in Form einer Öl-in-Wasser-Emulsion oder einer Wasser-in-Öl-Emulsion vorliegt, wobei die lyophilisierte Zusammensetzung nach Rehydratation wasserlöslich oder hydrodispersibel ist.

2. Artikel nach Anspruch 1, **dadurch gekennzeichnet, daß** das genannte Substrat eine poröse Struktur aufweist, die eine Imprägnierung der Zusammensetzung in das Substrat, wenigstens in der Nähe der Oberfläche des Substrats ermöglicht.

3. Artikel nach einem der Ansprüche 1 oder 2, **dadurch gekennzeichnet, daß** das Substrat ein Fasersubstrat ist.

4. Artikel nach Anspruch 3, **dadurch gekennzeichnet, daß** das Fasersubstrat von einer ungewebten Faserlage gebildet ist.

5. Artikel nach einem der Ansprüche 3 oder 4, **dadurch gekennzeichnet, daß** das Substrat aus Fasern synthetischer Herkunft, vorzugsweise aus Polyolefinen, Polyamiden, Polyestern oder Viskosen hergestellt ist.

6. Artikel nach einem der Ansprüche 1 bis 5, **dadurch gekennzeichnet, daß** das Substrat eine Dicke von 100 µm bis 3 cm, vorzugsweise von 0,2 mm bis 5 mm aufweist.

7. Artikel nach einem der Ansprüche 1 bis 6, **dadurch gekennzeichnet, daß** das Substrat eine ebene, flexible oder halbstarre Form hat.

8. Artikel nach einem der vorhergehenden Ansprüche, **dadurch gekennzeichnet, daß** das Substrat eine texturierte, geprägte, gerillte Oberfläche mit Vertiefungen und Buckeln oder ein gleichmäßiges, vorzugsweise durch gegenüber einer ebenen Oberfläche erhöhte Rippen oder Spitzen gebildetes Raster aufweist.

9. Artikel nach einem der vorhergehenden Ansprüche, **dadurch gekennzeichnet, daß** das Substrat eine Struktur mit größeren Oberflächenporen oder -vertiefungen von 100 µm bis 2 mm, wenigstens auf der Oberfläche, auf welcher die genannte Zusammensetzung aufgetragen wird, aufweist.

10. Artikel nach einem der vorhergehenden Ansprüche, **dadurch gekennzeichnet, daß** das Substrat vorzugsweise eine Form aufweist, die an die Form des Teils des Körpers angepaßt ist, auf das es aufgebracht werden soll; vorzugsweise ist der Artikel eine Maske und hat das Substrat eine angepaßte Form, um das Gesicht oder einen Teil des Gesichts, insbesondere die Augen, die Lippen, die Nase oder die Wangen zu bedecken.

11. Artikel nach einem der vorhergehenden Ansprüche, **dadurch gekennzeichnet, daß** der Strukturfestiger ein unvernetztes Hydrokolloid-Polymer natürlicher Herkunft ist.

12. Artikel nach Anspruch 11, **dadurch gekennzeichnet, daß** der Strukturfestiger ausgewählt ist aus:
- den natürlichen Strukturmitteln pflanzlicher Herkunft, wie Johannisbrotgummi, Guargummi, Pektine, Alginate, Carraghenane, Agar-Agar, Gummi Arabicum;
- den Mitteln mikrobieller Herkunft, wie Xanthangummi, Gellangummi, Hyaluronsäure, Dextran;
- den Mitteln tierischer Herkunft, wie Gelatine, Kollagen, Chitosan und dessen Derivate; und aus
- den halbsynthetischen Strukturmitteln, wie halbsynthetische oder synthetische Zellulose, wie Hydrogel-Polymere auf der Basis von nicht biologisch resorbierbaren Polymeren vom Typ Polyacryl oder Polyacrylamid.

13. Artikel nach Anspruch 12, **dadurch gekennzeichnet, daß** das hydrophile, lyophilisierte Strukturmittel ausgewählt ist aus der Hyaluronsäure, dem Chitosan und dessen Derivaten, den Alginaten oder dem Meereskollagen.

14. Artikel nach einem der vorhergehenden Ansprüche, **dadurch gekennzeichnet, daß** nach Rehydratation mit vorzugsweise 0,05 g/cm² bis 0,15 g/cm² Wasser, die genannte lyophilisierte, rehydratisierte Zusammensetzung eine Viskosität von 400 bis 5000 Zentipoise, vorzugsweise von 500 bis 2000 Zentipoise aufweist.

15. Artikel nach einem der vorhergehenden Ansprüche, **dadurch gekennzeichnet, daß** die lyophilisierte Zusammensetzung auf die Oberfläche des Substrats in einer Flächenmasse von 5 mg/cm² bis 1000 mg/cm², vorzugsweise von 20 mg/cm² bis 200 mg/cm² aufgebracht wird.

16. Artikel nach einem der vorhergehenden Ansprüche, **dadurch gekennzeichnet, daß** der wirksame Bestandteil ein hydrophiles oder lipophiles Mittel ist.

17. Artikel nach Anspruch 16, **dadurch gekennzeichnet, daß** die Zusammensetzung in Form einer Öl-in-Wasser-Emulsion oder einer Wasser-in-Öl-Emulsion vorliegt, wobei der wirksame Bestandteil in der Fettphase oder wäßrigen Phase gelöst ist.

18. Artikel nach Anspruch 17, **dadurch gekennzeichnet, daß** die Zusammensetzung in Form einer Öl-in-Wasser-Emulsion vorliegt, wobei der wirksame Bestandteil in der Fettphase der Emulsion fettlöslich ist, und daß die Zusammensetzung außerdem ein Netzhaftmittel enthält.

19. Artikel nach einem der Ansprüche 16 bis 18, **dadurch gekennzeichnet, daß** der wirksame Bestandteil ausgewählt ist aus einem Antioxidantium mit antiradikalischer Wirkung, einem depigmentierenden Mittel, einem Fettregulierungsmittel, einem Anti-Akne-Mittel, einem Mittel gegen übermäßige Absonderung der Talgdrüsen (Seborrhöe), einem Alterungsschutzmittel, einem lindernden Mittel, einem Antifaltenmittel, einem entzündungshemmenden Mittel, einem narbenbildenden Mittel, einem hydratisierenden Mittel, einem antibakteriellen Mittel, einem Mittel gegen Pilze, einem Vitamin, einem Protein, einer Aminosäure und einem ÖI.

20. Artikel nach einem der Ansprüche 16 bis 19, **dadurch gekennzeichnet, daß** der wirksame Bestandteil ausgewählt ist aus einem Öl, das reich an mehrfach ungesättigter Fettsäure ist, einem Phytosterol, einem Ceramid, einem Pflanzenextrakt, einem Algenextrakt oder einem Bakterienextrakt und einem Vitamin.

21. Artikel nach einem der Ansprüche 1 bis 20, **dadurch gekennzeichnet, daß** der Strukturfestiger eine hydratisierende Wirkung und der wirksame Bestandteil eine behandelnde Wirkung aufweist.

22. Artikel nach einem der Ansprüche 1 bis 21, **dadurch gekennzeichnet, daß** er in einem flexiblen oder halbstarren Tütchen oder in einer starren oder halbstarren Schale verpackt ist, wobei die Schale durch einen Deckel, vorzugsweise einen Metallfilm hermetisch verschlossen ist, wobei sich der Artikel in einer Vakuum- oder Neutralgasatmosphäre befindet.

23. Verfahren zur Herstellung eines Artikels nach einem der Ansprüche 1 bis 22, **dadurch gekennzeichnet, daß** die Schritte durchgeführt werden, bei denen:
- eine Lösung der genannten Zusammensetzung aus einem hydrophilen Strukturfestiger und vorzugsweise wenigstens einem wirksamen Bestandteil in flüssigem Zustand auf das genannte Substrat aufgebracht wird, anschließend
- die Zusammensetzung, sobald diese auf das Substrat aufgebracht ist, lyophilisiert wird.

24. Verfahren nach Anspruch 23, **dadurch gekennzeichnet, daß** eine wäßrige Lösung der Zusammensetzung mit einer Konzentration von 0,1 bis 5, vorzugsweise von 0,5 bis 2 Gewichtsprozent des hydrophilen Strukturfestigers verwendet wird, wobei dieser vorzugsweise eine Viskosität von 200 bis 5000 Zentipoise, vorzugsweise von 400 bis 1000 Zentipoise aufweist.

25. Verfahren nach einem der Ansprüche 23 oder 24, **dadurch gekennzeichnet, daß** die Schritte durchgeführt werden, bei denen:
- das Substrat zuvor zugeschnitten wird, vorzugsweise entsprechend einer geometrischen Form, die in Abhängigkeit von dem Bereich des Körpers definiert ist, für den der Artikel bestimmt ist,
- das Substrat und die Matrix in eine Tragschale eingebracht werden,
- die Lyophilisation des Artikels in der Schale vollzogen wird.

26. Verfahren nach Anspruch 25, **dadurch gekennzeichnet, daß** die Schale als Endverpackung des Artikels verwendet wird, wobei die Schale mit einem Deckel versehen wird, wobei sich der Artikel in einer Vakuum- oder Neutralgasatmosphäre befindet.

## Claims

1. Cosmetic or dermatological product suitable for skin application, comprising a freeze-dried composition having cosmetic or dermatological action adhering to a solid substrate, **characterized in that** said freeze-dried composition comprises a matrix with a porous expanded structure of a hydrophilic texturing agent that is a non-crosslinked hydrocolloid polymer preferably containing at least one active ingredient, said composition being in the form of an oil-in-water emulsion or water-in-oil emulsion, said freeze-dried composition being water-soluble or water-dispersible after re-hydration.

2. Product as in claim 1, **characterized in that** said substrate has a porous structure enabling impregnation of said composition in said substrate, at least in the vicinity of the surface of said substrate.

3. Product as in either of claims 1 or 2, **characterized in that** said substrate is a fibrous substrate.

4. Product as in claim 3, **characterized in that** said fibrous substrate consists of a non-woven fibrous sheet.

5. Product as in either of claims 3 or 4, **characterized in that** the substrate is made from fibres of synthetic origin, preferably polyolefins, polyamides, polyesters or viscose.

6. Product as in any of claims 1 to 5, **characterized in that** the said substrate has thickness of 100 µm to 3 cm, preferably of 0.2 mm to 5 mm.

7. Product as in any of claims 1 to 6, **characterized in that** said substrate is of flexible or semi-rigid planar shape.

8. Product as in any of the preceding claims, **characterized in that** said substrate has a grooved, goffered, textured surface with depressions and embossments, or a regular weft preferably consisting of ribs or raised dots with respect to a planar surface.

9. Product as in any of the preceding claims, **characterized in that** said substrate has a texture with surface pores or hollows of larger size 100 µm to 2 mm at least on a surface on which said composition is applied.

10. Product as in any of the preceding claims, **characterized in that** preferably said substrate has a shape adapted to the shape of that part of the body to which it is intended to be applied; preferably said product is a mask and said substrate has a shape adapted to cover the face or part of the face, in particular the eyes, lips, nose or cheeks.

11. Product as in any of the preceding claims, **characterized in that** said texturing agent is a non crosslinked hydrocolloid polymer of natural origin.

12. Product as in claim 11, **characterized in that** said texturing agent is chosen from among:
- natural texturing agents of plant origin such as carob gum, guar gum, pectins, alginates, carrageenans, agar-agar, gum arabic;
- agents of microbial origin such as xanthan gum, gellan gum, hyaluronic acid, dextran;
- agents of animal origin such as gelatin, collagen, chitosan and its derivatives, and
- semi-synthetic texturing agents such as semi-synthetic or synthetic cellulose such as non-bioresorbable hydrogel polymers of polyacrylic or polyacrylamide type.

13. Product as in claim 12, **characterized in that** the freeze-dried hydrophilic texturing agent is chosen from among hyaluronic acid, chitosan and its derivatives, alginates or marine collagen.

14. Product as in any of the preceding claims, **characterized in that**, after re-hydration with preferably 0.05 g/cm² to 0.15 g/cm² water, said re-hydrated, freeze-dried composition has a viscosity of 400 to 5000 centipoises, preferably 500 to 2000 centipoises.

15. Product as in any of the preceding claims, **characterized in that** said freeze-dried composition is applied to the surface of said substrate with a gram weight of 5 mg/cm² to 1000 mg/cm², preferably 20 mg/cm² to 200 mg/cm².

16. Product as in any of the preceding claims, **characterized in that** said active ingredient is a hydrophilic or lipophilic agent.

17. Product as in claim 16, **characterized in that** said composition is in the form of an oil-in-water emulsion or a water-in-oil emulsion, said active ingredient being dissolved in the fat or aqueous phase.

18. Product as in claim 17, **characterized in that** said composition is in the form of an oil-in-water emulsion, said active ingredient being liposoluble in said fat phase of the emulsion, and said composition also containing a surfactant.

19. Product as in any of claims 16 to 18, **characterized in that** said active ingredient is chosen from among an anti-oxidizing agent having anti-radical action, a depigmenting agent, a liporegulating agent, an anti-acne agent, an anti-seborrhoea agent, an anti-ageing agent, a softening agent, an anti-wrinkle agent, an anti-inflammatory agent, a healing agent, a hydrating agent, an anti-bacterial agent, an antifungal agent, a vitamin, a protein, an amino acid and an oil.

20. Product as in any of claims 16 to 19, **characterized in that** said active ingredient is chosen from among an oil rich in polyunsaturated fatty acid, a phytosterol, a ceramide, a plant extract, an algae extract or a bacterial extract and a vitamin.

21. Product as in any of claims 1 to 20, **characterized in that** said texturing agent has a hydrating action and said active ingredient has a treatment action.

22. Product as in any of claims 1 to 21, **characterized in that** it is packaged in a flexible or semi-rigid pouch or in a rigid or semi-rigid shell, said shell being hermetically sealed by a film, preferably a metal film, the product being in a vacuum or neutral gas atmosphere.

23. Method for manufacturing a product as in any of claims 1 to 22, **characterized in that** it comprises the steps in which:
- a solution of said composition of a hydrophilic texturing agent and preferably of at least one active ingredient in liquid state is applied onto said substrate, then
- said composition is freeze-dried after it has been applied onto said substrate.

24. Method as in claim 23, **characterized in that** an aqueous solution of said composition is used at a concentration of 0.1 to 5 % preferably 0.5 to 2 % by weight of said hydrophilic texturing agent, said agent preferably having a viscosity of 200 to 5000 centipoises, preferably 400 to 1000 centipoises.

25. Method as in either of claims 23 or 24, **characterized in that** the steps are performed in which:
- said substrate is pre-cut, preferably according to a geometric shape in relation to the area of the body for which the product is intended,
- said substrate and said matrix are inserted in a carrier shell,
- the freeze-drying of said product is performed in said shell.

26. Method as in claim 25, **characterized in that** said shell is used as end packaging for the product, said shell being coated with a film, said product being in a vacuum or neutral gas atmosphere.
